# EUROPÄISCHE PATENTSCHRIFT

(11) **EP 2 567 724 B1**
(45) Veröffentlichungstag und Bekanntmachung des Hinweises auf die Patenterteilung: **12.08.2015**
(21) Anmeldenummer: 12195455.6
(22) Anmeldetag: 02.02.2007
(51) Int. Cl.: A61M 16/10, A61M 16/14, A61M 16/16, A61M 16/00, A61M 16/04, A61M 11/00

(54) **Inhalationstherapievorrichtung**
Inhalation therapy device
Dispositif de thérapie par inhalation

(30) Priorität: 10.02.2006 DE 102006006183
(43) Veröffentlichungstag der Anmeldung: 13.03.2013
(62) Teilanmeldung aus: 07002327.0
(73) Patentinhaber: PARI Pharma GmbH, 82319 Starnberg (DE)
(72) Erfinder: Minocchieri, Dr. Stefan, 8197 Rafz (CH); Gallem, Thomas, 81371 München (DE); Vogelmann, Martina, 82407 Wielenbach (DE)
(74) Vertreter: Hoffmann Eitle

(56) Entgegenhaltungen:
- WO-A2-01/41854
- WO-A2-2005/048982
- WO-A2-2005/102431
- US-A- 5 474 058
- US-A1- 2003 072 717
- US-A1- 2004 011 364
- US-B1- 6 729 334

## Beschreibung

Die vorliegende Erfindung betrifft eine Inhalationstherapievorrichtung für die Anwendung bei Frühgeborenen und Kleinkindern.

Frühgeborene Kinder unter 34 Schwangerschaftswochen leiden an einem Surfactant-Mangelsyndrom. Synonyme für diese Krankheit sind: HMK (Hyaline-Membrankrankheit), Atemnotsyndrom des Frühgeborenen, IRDS (Infant Respiratory Distress Syndrome). Die Surfactant-Substitutionstherapie ist bereits gut etabliert und gehört zu den Standardtherapieverfahren in der Neonatologie (Frühgeborenen- und Neugeborenenmedizin). Um den Umfang des Einsatzbereichs einer erfindungsgemäßen Inhalationstherapievorrichtung anzudeuten, sei daraufhingewiesen, dass in der Schweiz pro Jahr ca. 550 Kinder vor Erreichen der 35. Schwangerschaftswoche geboren werden und damit potenziell eine unreife Lunge aufweisen, bei der eine Surfactant-Substitutionstherapie angezeigt ist. In anderen Ländern, beispielsweise Deutschland, kann mit einer zehnfach höheren Zahl frühgeborener Kinder gerechnet werden.

Die Surfactant-Substitutionstherapie erfolgt, während sich die Frühgeborene/Kleinkinder in sogenannten Brutkästen befinden, das heißt in einer Umgebung mit kontrollierter Temperatur und Luftfeuchte, da die Frühgeborenen ihre Körpertemperatur noch nicht selbst aufrecht erhalten können. Das Surfactant wird über einen Tubus direkt in die Trachea in flüssiger Form instilliert. Die Intubation an sich birgt verschiedene Risiken, zum Beispiel Verletzung der Glottis oder der Luftröhre, Pneumothorax etc. Überdies kann die maschinelle Beatmung, die in der Regel mit der Instillation einhergeht, zu zusätzlichen Schädigungen der Lunge führen. Viele der Frühgeborenen/Kleinkinder haben jedoch einen ausreichenden eigenen Atemantrieb und müssten vor diesem Hintergrund nicht intubiert werden. Um jedoch das Surfactant in der Lunge zu deponieren, ist die Intubation das Mittel der Wahl für die Instillation des Surfactants.

Während die Surfactant-Substitutionstherapie intensiv untersucht wurde und bereits umfangreich eingesetzt wird, ist die Verneblung des Surfactants problembehaftet, da das Surfactant regelmäßig eine geringe Oberflächenspannung, eine für die Vernebelung ungünstige Viskosität und eine Neigung zur Schaumbildung aufweist. Die physikalischen Eigenschaften des Surfactants haben dazu geführt, dass an eine Verneblung und die Applikation des Surfactants in Form eines Aerosols praktisch nicht gedacht wurde. Zudem ist ein Surfactant in der Regel sehr teuer, so dass die bei Aerosoltherapie oftmals zu beobachtenden hohen Depositionsverluste dazu geführt haben, dass diese Art der Applikation eines Surfactants nicht weiter untersucht wurde.

Eine Inhalationstherapievorrichtung mit den Merkmalen im Oberbegriff des Anspruchs 1 ist aus der WO-A-2005/048982 bekannt. Ferner offenbart die US-A-2003/0072717 einen Inhalator mit einem zylindrischen Gehäuse mit konstantem Durchmesser, in dem eine Aerosolerzeugungseinrichtung aufgenommen ist. Die Strömung durch das Gehäuse wird beim Einatmen des Patienten durch dessen Spontanatmung erzeugt. Durch den konstanten Durchmesser ist diese Strömung laminar.

Vor diesem Hintergrund strebt die vorliegende Erfindung an, einen Weg aufzuzeigen, wie Surfactant bei Frühgeborenen und Kleinkindern im Rahmen einer Aerosoltherapie appliziert werden kann.

Dieses Ziel wird erreicht durch eine Inhalationstherapievorrichtung mit den Merkmalen in Anspruch 1.

Die Erfindung bringt für den speziellen Einsatzbereich drei wesentliche Aspekte zusammen, nämlich die präzise Erzeugung eines für die Applikation, bei Frühgeborenen und Kleinkindern, besonders geeigneten Aerosols, die dem CPAP/BIPAP-Prinzip folgende Aufbringung eines leichten (ggf. pulsatilen) Überdrucks in die Atemwege/Lunge sowie die weitgehend verlustfreie Zuführung eines Aerosols über eine verjüngend zulaufende Vernebelungskammer und die mantelförmige Umströmung der erzeugten Flüssigkeitströpfchen. Dabei ist ferner zu berücksichtigen, dass vorteilhafterweise aufgrund der insgesamt sehr gering realisierbaren Abstände und Abmessungen in Bezug auf die Vernebelungskammer nur ein sehr kleines Totvolumen vorhanden ist. Damit steht das zu verabreichende Aerosol sehr früh zu Beginn eines Atemzyklus zur Verfügung und gelangt tief in die Atemwege und Lungen.

Grundsätzlich eignet sich eine erfindungsgemäße Inhalationstherapievorrichtung damit aber auch für andere Anwendungszwecke.

Weiterbildungen der Erfindung finden sich in den Unteransprüchen.

Weitere Aspekte können, wie sich aus der weiter unten stehenden Beschreibung ergibt, hinzutreten, um die Wirksamkeit und Effektivität zu verbessern. In diesem Zusammenhang ist insbesondere auf die Erwärmung und die Befeuchtung der Atemluft, auf das Aufbringen einer Druckschwingung auf den Atemluftstrom und auf das Erwärmen der zu vernebelnden Flüssigkeit hinzuweisen.

Im Folgenden wird die Erfindung anhand von Ausführungsbeispielen genauer erläutert. Dabei wird auf die Figuren Bezug genommen, in denen zeigt:
- Figur 1: ein erstes Ausführungsbeispiel einer erfindungsgemäßen Inhalationstherapievorrichtung, mit den grundlegenden Komponenten;
- Figur 1a: eine vergrößerte Ansicht eines in besonderer Weise ausgestalteten Teils des Ausführungsbeispiels gemäß Figur 1;
- Figur 2: ein zweites Ausführungsbeispiel einer erfindungsgemäßen Inhalationstherapievorrichtung mit einer Atemluftheizeinrichtung;
- Figur 3: ein drittes Ausführungsbeispiel einer erfindungsgemäßen Inhalationstherapievorrichtung mit einer Atemluftbefeuchtungseinrichtung;
- Figur 4: ein viertes Ausführungsbeispiel einer erfindungsgemäßen Inhalationstherapievorrichtung mit einer Atemluftstrompulsationseinrichtung; und
- Figur 5: ein fünftes Ausführungsbeispiel einer erfindungsgemäßen Inhalationstherapievorrichtung mit mehreren Zusatzeinrichtungen und einer Steuereinrichtung.

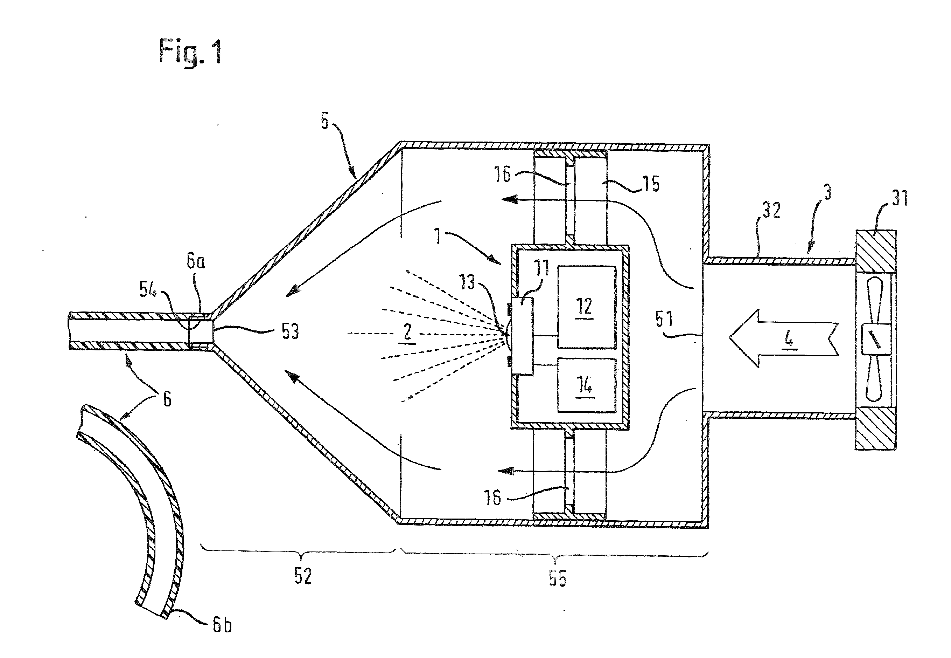

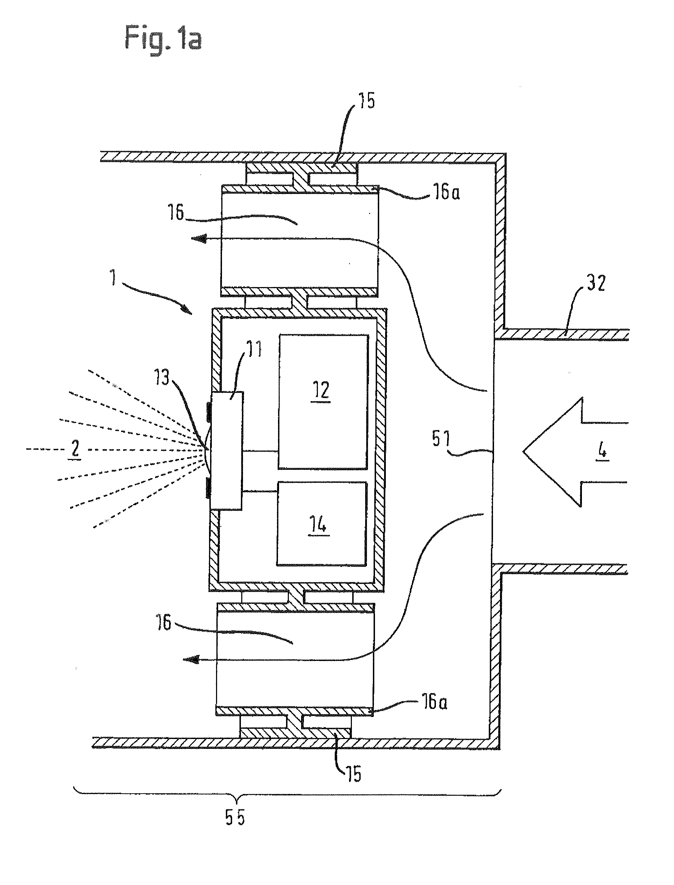

Bei dem in Figur 1 gezeigten Ausführungsbeispiel einer erfindungsgemäßen Inhalationstherapievorrichtung ist eine Aerosolerzeugungseinrichtung 1 für die Vernebelung eines Fluids und die Bereitstellung von Flüssigkeitströpfchen 2 vorgesehen. Die schematisch dargestellte Aerosolerzeugungseinrichtung 1 umfasst in der gezeigten Ausgestaltung einen Aerosolgenerator 11, dem eine Flüssigkeit zugeführt wird, die in einem Reservoir 12 bevorratet wird. In der gezeigten Ausgestaltung umfasst der Aerosolgenerator 11 eine Membran 13, mit deren Hilfe die aus dem Reservoir zugeführte Flüssigkeit vernebelt wird, so dass der Aerosolgenerator 11 eine definierte Menge von Flüssigkeitströpfchen 2 abgibt. Der Aerosolgenerator 11 wird von einer Aerosolerzeugungssteuerung 14 der Aerosolerzeugungseinrichtung 1 gesteuert.

Vor dem Hintergrund des Einsatzes bei Frühgeborenen und Kleinkindern liegt die Größe der Flüssigkeitströpfchen (MMD) bei einer erfindungsgemäßen Inhalationstherapievorrichtung zwischen 1,5 und 3 µm. Die Einhaltung dieser Vorgaben ist mit besonders hoher Genauigkeit bei einer Aerosolerzeugungseinrichtung 1 möglich, die einen zuvor bereits angesprochenen Aerosolgenerator 11 mit einer Membran 13 für die Erzeugung der Flüssigkeitströpfchen aufweist. Damit stellt eine Aerosolerzeugungseinrichtung mit Membran-Aerosolgenerator eine bevorzugte Ausführungsform der Erfindung dar.

Das in Figur 1 gezeigte Ausführungsbeispiel einer erfindungsgemäßen Inhalationstherapievorrichtung umfasst ferner eine Einrichtung 3 zur Erzeugung einer Atemluftströmung 4. Dazu umfasst die Atemluftstromerzeugungseinrichtung 3 beispielsweise einen Ventilator 31, der den Atemluftstrom 4 erzeugt und in eine Zuführleitung 32 hineinfördert. Durch eine entsprechende Steuerung der Atemluftstromerzeugungseinrichtung 3 sind Druck und Fluss der Atemluftströmung 4 an die spezifische Therapiesituation anpassbar.

Bei Frühgeborenen und Kleinkindern sind ein Druck von maximal 4 bis 7 mbar und ein Atemvolumen von etwa 5 ml pro kg Körpergewicht als geeignete Vorgabe anzusetzen. Bei diesen Vorgaben erfolgt die Ventilation der Frühgeborenen/Kleinkinder vor dem Hintergrund der eigenen Atmung entsprechend dem CPAP-Prinzip (Continuous Positive Airway Pressure). Voraussetzung für die Anwendung einer CPAP-Ventilation ist stets die Fähigkeit des Patienten zur Atmung, wobei aber bei Frühgeborenen und Kleinkindern durch den CPAP-Überdruck vorteilhafterweise erreicht wird, dass die Lungen etwas vorgebläht werden und ein Kollabieren bereits belüfteter Alveolen verhindert wird. Andere Verfahren, wie zum Beispiel nach dem BIPAP-Prinzip (Biphasic Positive Airway Pressure) sind ebenfalls anwendbar. Die anwendbaren Drücke richten sich nach den konkreten Gegebenheiten und können Werte von 10 mbar (CPAP) und 15 mbar (BIPAP) erreichen und sogar überschreiten.

Das in Figur 1 gezeigte Ausführungsbeispiel einer erfindungsgemäßen Inhalationstherapievorrichtung weist ferner eine Vernebelungskammer 5 auf, in die sowohl die Flüssigkeitströpfchen 2, die von der Aerosolerzeugungseinrichtung 1 erzeugt werden, als auch die Atemluftströmung 4, die von der Atemluftstromerzeugungseinrichtung 3 erzeugt wird, zugeführt werden. Bei dem in Figur 1 gezeigten Ausführungsbeispiel geschieht dies im Hinblick auf die Flüssigkeitströpfchen 2 dadurch, dass die Aerosolerzeugungseinrichtung 1 in der Vernebelungskammer 5 angeordnet ist, so dass die Abgabe der Flüssigkeitströpfchen 2 unmittelbar in der Vernebelungskammer 5 erfolgt. Diese Anordnung der Aerosolerzeugungseinrichtung 1 führt auch zu einer Gestaltung des erfindungsgemäßen Inhalationstherapiesystems, bei dem Totvolumina der Vernebelungskammer 5 auf ein Minimum reduziert werden können.

Die Zuführung der Atemluftströmung 4 erfolgt durch eine Atemluftzuführöffnung 51 der Vernebelungskammer 5, an der die Zuführleitung 32 der Atemluftstromerzeugungseinrichtung 3 angeordnet ist. Die Zuführung erfolgt vorzugsweise derart, dass sich beim Eintreten der Atemluft 4 in die Vernebelungskammer 5 eine verwirbelte Strömung ausbildet. Auf diese Weise wird sichergestellt, dass nur minimale Toträume der Vernebelungskammer 5 in der Nähe der Atemluftzuführöffnung 51 unter Umständen nicht durchströmt werden, wodurch eine Versorgung des Frühgeborenen/Neugeborenen mit sauerstoffhaltiger Frischluft bestmöglich gewährleistet wird. In der Regel wird aber die verwirbelte Strömung eine vollständige oder weitgehend vollständige Durchströmung der Vernebelungskammer 5 gewährleisten. Wie weiter unten noch erläutert wird, sind in einer bevorzugten Ausgestaltung der Erfindung Mittel vorgesehen, die die verwirbelte Strömung in eine weitgehend gerichtete Strömung überführen.

Die Flüssigkeitströpfchen 2 und die Atemluft 4 vermischen sich in der Verneblungskammer 5 und gelangen aufgrund der durch die Atemluft 4 vorgegebenen Strömung, wie Figur 1 zeigt, in einen sich verjüngenden Bereich 52 der Vernebelungskammer 5, der in eine Austrittsöffnung 53 mündet. Durch die Öffnung 53 der Vernebelungskammer 5 kann das Flüssigkeitströpfchen/Atemluft-Gemisch, das durch Zuführen der Flüssigkeitströpfchen 2 und der Atemluft 4 in die Vernebelungskammer 5 gebildet wurde, aus der Vernebelungskammer 5 austreten.

Das in Figur 1 gezeigte Ausführungsbeispiel weist eine schlauchförmige Intubierungseinrichtung 6 auf, deren erstes Ende 6a für den Anschluss an die Austrittsöffnung 53 der Vernebelungskammer 5 ausgebildet ist. Vorzugsweise ist das erste Ende 6a der Intubierungseinrichtung 6 auf einen Stutzen 54 der Vernebelungskammer 5 aufsteckbar, der an der Austrittsöffnung 53 vorgesehen ist. Durch die Anbringung der schlauchförmigen Intubierungseinrichtung 6 gemäß der Erfindung tritt das Flüssigkeitströpfchen/Atemluft-Gemisch aus der Vernebelungskammer 5 durch die Austrittsöffnung 53 in die schlauchförmige Intubierungseinrichtung 6 ein, durchströmt diese und gelangt zum zweiten Ende 6b der schlauchförmigen Intubierungseinrichtung 6.

Um einen weitgehend depositionsfreien Transport des Flüssigkeitströpfchen/Atemluft-Gemisches durch die Intubierungseinrichtung zu gewährleisten, wird zweckdienlicherweise ein Schlauch mit 2 bis 3,5 mm Innendurchmesser eingesetzt. Ebenfalls zur Minimierung von Depositionsverlusten sollte die Gesamtlänge der schlauchförmigen Intubierungseinrichtung 50 cm nicht übersteigen.

Überraschenderweise stellen sich bei Einhaltung dieser Vorgaben sehr gute Ergebnisse ein, die so angesichts der vergleichsweise klein erscheinenden Passagen für das Aerosol nicht erwartet werden konnten. Dies gilt umso mehr für ein Surfactant als zu vernebelnde Flüssigkeit, deren physikalische Eigenschaften nicht erahnen ließen, dass bei Einhaltung bestimmter Vorgaben und geeigneter Vernebelung eine Aerosol-Applikation von Surfactant bei Frühgeborenen und Kleinkindern auf dem Wege der Inhalation möglich ist.

Das zweite Ende der Intubierungseinrichtung 6 ist für eine endotracheale/endopharyngeale Intubation ausgestaltet, wobei die Ausgestaltung vorteilhaft derart erfolgt, dass das zweite Ende bei einer orotrachealen Intubation durch den Mund hinter den Stimmlippen eines Patienten und bei einer nasophayngealen Intubation durch die Nase hinter dem Nasenraum im Rachen eines Patienten positionierbar ist. Durch die erfindungsgemäße Positionierbarkeit des zweiten Endes 6b der schlauchförmigen Intubierungseinrichtung 6 wird sichergestellt, dass das durch die Intubierungseinrichtung geförderte Flüssigkeitströpfchen/Atemluft-Gemisch hinter die jeweils stark filternden Bereiche des Atemwegs des Patienten gelangt. Im Fall einer Applikation durch die Nase ist es erforderlich, den Nasenbereich zu überbrücken und das Flüssigkeitströpfchen/Atemluft-Gemisch in den Rachen des Patienten abzugeben, wohingegen bei einer Applikation über den Mund die Abgabe des Flüssigkeitströpfchen/Atemluft-Gemisches vorzugsweise hinter der Glottis erfolgt. Bei der Ausgestaltung des Intubationsendes 6b liegt das Hauptaugenmerk demzufolge auf der Länge dieses Bereichs, da die Länge des zweiten Endes 6b der Intubierungseinrichtung 6 festlegt, an welchem Punkt der Patientenatemwege die Abgabe des Flüssigkeitströpfchen/Atemluft-Gemisches (des Aerosols) erfolgt. Bei Frühgeborenen und Kleinkindern ist eine Länge von ca. 15 cm zweckdienlich.

Im Zusammenwirken der einzelnen Komponenten der in Figur 1 gezeigten erfindungsgemäßen Inhalationstherapievorrichtung wird erreicht, dass die Flüssigkeitströpfchen 2 und die Atemluftströmung 4 in der Vernebelungskammer 5 zusammengeführt werden, sich dort vermischen und als Flüssigkeitströpfchen/Atemluft-Gemisch, das heißt als Aerosol, über den verjüngten Bereich 52 der Vernebelungskammer in die Intubierungseinrichtung 6 gefördert werden, um erst hinter den stark filternden Atemwegsbereichen des Patienten aus der Intubierungseinrichtung abgegeben zu werden. Damit erzielt die erfindungsgemäße Inhalationstherapievorrichtung eine besonders wirksame Applikation der vernebelten Flüssigkeit, so dass sich für die erfindungsgemäße Inhalationstherapievorrichtung Anwendungsgebiete erschließen, die bei herkömmlichen Vorrichtungen aufgrund mangelhafter Applikationsgenauigkeit und -effektivität erreichbar waren.

So ist, wie eingangs angesprochen, mit Hilfe einer erfindungsgemäßen Inhalationstherapievorrichtung die Verabreichung von Surfactant bei Frühgeborenen und Kleinkindern möglich. Surfactant reduziert die Oberflächenspannung in den Alveolen und erleichtert somit den Kindern die Atemarbeit, was zu einer verbesserten Sauerstoffaufnahme führt. Die positive Wirkung einer Behandlung mit Surfactant ist in der Literatur vielfach beschrieben. Unbekannt ist jedoch die Applikation eines Surfactant-Aerosols, das mit Hilfe der erfindungsgemäßen Inhalationstherapievorrichtung appliziert werden kann. Diese Möglichkeit wird durch die Erfindung geschaffen, da das mehrere Aspekte umfassende Gesamtkonzept der erfindungsgemäßen Inhalationstherapievorrichtung dazu führt, dass eine Therapievorrichtung zur Verfügung steht, die die Applikation eines Surfactants auf diese Weise ermöglicht.

Zu dem in Figur 1 gezeigten Ausführungsbeispiel ist anzumerken, dass die Anordnung der Aerosolerzeugungseinrichtung 1 im Inneren der Vernebelungskammer 5 besonders vorteilhaft ist, da auf diese Weise eine unerwünschte Deposition der Flüssigkeitströpfchen in der Vernebelungskammer 5 minimiert wird. Die Anordnung trägt außerdem zur Minimierung von Totraumvolumina in der Vernebelungskammer 5 bei. Überdies kann die Anordnung des Aerosolgenerators 11 der Aerosolerzeugungseinrichtung 1 so erfolgen, dass sie bezogen auf den verjüngten Bereich 52 der Vernebelungskammer in geeigneter Weise ausgerichtet ist. Bei einer grundsätzlich rotationssymmetrischen Gestaltung der erfindungsgemäßen Inhalationstherapievorrichtung, wie sie in Figur 1 angedeutet ist, bedeutet dies, dass der Aerosolgenerator 11 der Aerosolerzeugungseinrichtung 1 auf der Rotationsachse derart angeordnet ist, dass die Flüssigkeitströpfchen sich entlang der Rotationsachse auf den sich verjüngenden Bereich 52 zu bewegen. Damit ist die erfindungsgemäße Vorrichtung auch in beliebigen Lagen in Bezug auf die Rotationsachse betreibbar. Auch die Zuführung der Atemluft 4 erfolgt dann entlang der Rotationsachse, so dass sich eine um die Aerosolerzeugungseinrichtung herum laufende Atemluftströmung einstellt, die in Figur 1 durch entsprechende Pfeile angedeutet ist. Damit die Atemluft 4 die Aerosolerzeugungseinrichtung 1 umströmen kann, weist eine Halterungseinrichtung 15 der Aerosolerzeugungseinrichtung 1 Durchtrittsöffnungen 16 auf, damit die Atemluft 4 die Aerosolerzeugungseinrichtung 1 in der Vernebelungskammer 5 praktisch ungehindert umströmen kann. Jedoch bewirken die Durchtrittsöffnungen 16 in vorteilhafter Weise, dass eine sich nach dem Eintritt durch die Atemluftzuführöffnung verwirbelt ausbildende Atemluftströmung in eine weitgehend laminare Strömung umgewandelt wird, die den von Trägheitskräften weitestgehend unbeeinflussten Transport der zuströmenden Flüssigkeitströpfchen begünstigt. Im Übrigen weist die Halterungseinrichtung 15 aber eine ausreichende Anzahl von radial verlaufenden Stützelementen auf, so dass die Halterungseinrichtung 15 die Aerosolerzeugungseinrichtung 1 in dem dafür vorgesehenen Bereich 55 der Vernebelungskammer 5 fixiert.

Um die Umwandlung der Atemluftströmung in eine weitestgehend laminare Strömung zu unterstützen, können die Durchtrittsöffnungen 16 mit Atemluftführungen 16a ausgestattet werden, wie Figur 1a zeigt. Vorteilhafterweise sind die Atemluftführungen kurze rohrförmige Elemente 16a, die beispielsweise einen kreisförmigen Querschnitt aufweisen.

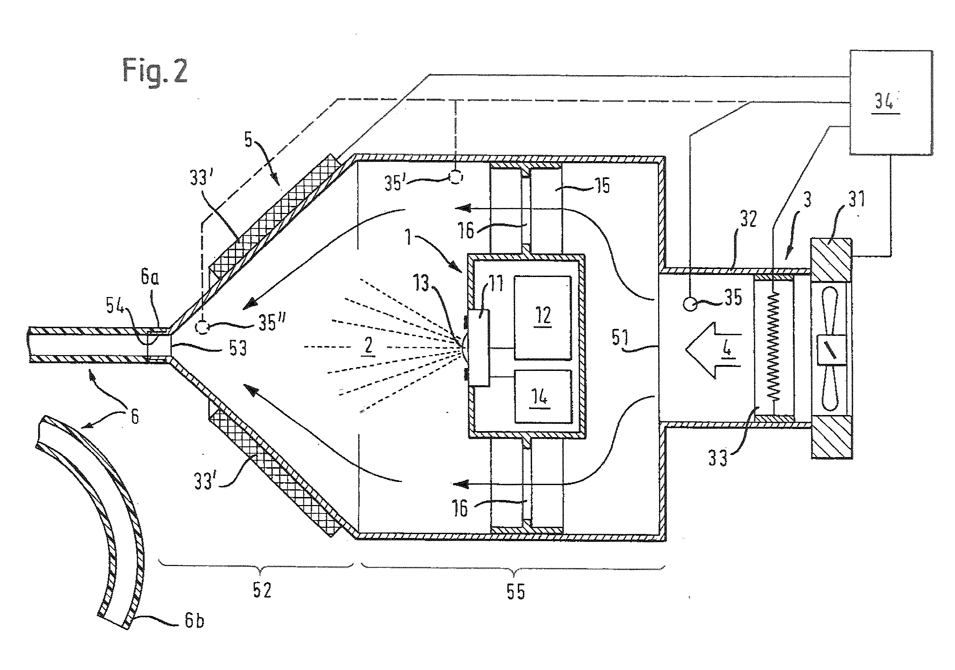

Figur 2 zeigt ein weiteres Ausführungsbeispiel einer erfindungsgemäßen Inhalationstherapievorrichtung, das hinsichtlich der Grundkomponenten mit dem ersten Ausführungsbeispiel übereinstimmt. Insofern wird auf die vorangegangene Beschreibung Bezug genommen; Figur 2 weist demzufolge auch dieselben Bezugszeichen auf, die in Figur 1 verwendet wurden.

Bei dem zweiten Ausführungsbeispiel ist, wie in Figur 2 gezeigt, eine Atemluftheizvorrichtung 33 vorgesehen, die die Atemluft 4 erwärmt, die von der Atemluftstromerzeugungseinrichtung 3 in die Vernebelungskammer 5 gefördert wird. Für die Regelung der Atemlufttemperatur ist eine Atemluftsteuereinrichtung 34 vorgesehen, die die Atemluftheizeinrichtung 33 auf der Basis eines Messsignals steuert, das von einem Temperatursensor 35 bereitgestellt wird. Der Sensor 35 ist in der Strömung der erwärmten Atemluft 4 angeordnet, beispielsweise in der Zuführleitung 32 der Atemluftstromerzeugungseinrichtung 3. Alternativ kann, wie in Figur 2 gezeigt, der Atemluftsensor 35', 35" auch an einer geeigneten Stelle in oder an der Vernebelungskammer 5 angeordnet sein. In einer besonders bevorzugten Ausgestaltung erfolgt die Temperaturmessung durch einen Sensor 35" in der Nähe der Austrittsöffnung 53 der Vernebelungskammer 5. Jedoch können auch zwei oder mehr Sensoren eingesetzt werden, um beispielsweise die Temperatur der Atemluft im Bereich der Zuführleitung 32 einerseits und im Bereich der Austrittsöffnung 53 andererseits zu erfassen und bei der Steuerung der Atemluftheizeinrichtung 33 durch die Atemluftsteuereinrichtung 34 zu berücksichtigen.

Die Atemluftheizeinrichtung 33 wird vorzugsweise durch die Atemluftsteuereinrichtung 34 derart angesteuert, dass die über die Intubiereinrichtung dem Patienten zugeführte Atemluft auf 35°C bis 37°C erwärmt wird. Die Atemluftsteuereinrichtung 34 regelt die Temperatur der Atemluft unabhängig von den äußeren Bedingungen in einem engen Bereich. Dabei kann die Atemluftsteuereinrichtung 34 mit der Luftfördereinrichtung 31 verbunden sein und deren Ventilator unter Berücksichtigung der Messwerte steuern, die von den Messsensoren 35, 35' und/oder 35" zur Atemluftsteuereinrichtung 34 geliefert werden.

Schließlich kann eine Verbindung zwischen der Aerosolerzeugungseinrichtung 1, genauer deren Steuereinrichtung 14 und der Atemluftsteuereinrichtung 34 zweckdienlich sein, damit der Beginn der Vernebelung der Flüssigkeit bei der Ansteuerung der Atemluftheizeinrichtung 33 berücksichtigt wird. Denn durch die Erzeugung der Flüssigkeitströpfchen 2 in der Vernebelungskammer 5 tritt eine Abkühlung der in der Vernebelungskammer 5 vorhandenen Atemluft auf, die sich mit den Flüssigkeitströpfchen durchmischt, da die Flüssigkeitströpfchen durch die Atemluft abgetrocknet werden. Dieser Abtrocknungseffekt ist grundsätzlich wünschenswert, da dadurch Einfluss genommen werden kann auf die Größe der Flüssigkeitströpfchen, so dass eine genauere Einhaltung der Vorgaben (siehe oben) möglich ist. Durch eine entsprechende Steuerung der Heizeinrichtung 33 kann somit letztlich die Temperatur und die Tröpfchengröße (durch kontrollierte Abtrocknung) des Aerosols festgelegt werden, insbesondere dann, wenn ein Sensor 35" in der Nähe der Austrittsöffnung 53 der Vernebelungskammer 5 eingesetzt wird. Unterstützend kann eine Erwärmungseinrichtung für die in dem Aerosolerzeuger bevorratete Flüssigkeit vorgesehen werden, die die bevorratete Flüssigkeit erwärmt. Vorzugsweise wird die Erwärmungseinrichtung von der Steuereinrichtung 14 gesteuert.

Als Alternative oder zusätzlich zu der in Figur 2 gezeigten Atemluftheizeinrichtung 33 kann in einer weiteren Ausgestaltung der erfindungsgemäßen Inhalationstherapievorrichtung eine Aerosolheizeinrichtung 33' vorgesehen werden, die vorzugsweise an der Vernebelungskammer 5 angeordnet ist, wie auch Figur 2 zeigt. Die Aerosolheizeinrichtung 33' erwärmt das Aerosol durch IR-Strahlung. Auch die Aerosolheizeinrichtung 33' ist vorteilhafterweise an die Atemluftsteuereinrichtung 34 angeschlossen und wird wie zuvor für die Heizeinrichtung 33 beschrieben in die Steuerung einbezogen. Auf die vorstehende Beschreibung wird insofern Bezug genommen.

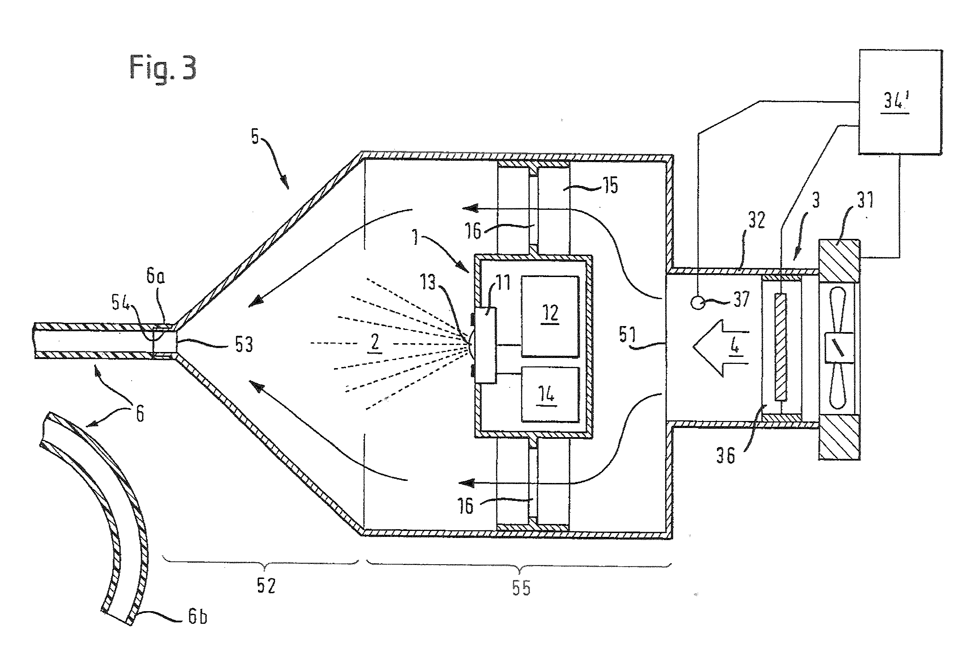

Figur 3 zeigt ein weiteres Ausführungsbeispiel einer erfindungsgemäßen Inhalationstherapievorrichtung. Ebenso wie bei Figur 2 stimmt der Aufbau des Ausführungsbeispiels gemäß Figur 3 im Wesentlichen mit dem Aufbau des ersten Ausführungsbeispiels überein. Dementsprechend sind die Bezugszeichen aus Figur 1 auch in Figur 3 vorhanden.

In Abwandlung des ersten Ausführungsbeispiels umfasst die erfindungsgemäße Inhalationstherapievorrichtung gemäß Figur 3 eine Atemluftbefeuchtungseinrichtung 36, die in Bezug auf die Atemluftströmung 4 derart angeordnet ist, dass sie die von der Atemluftströmerzeugungseinrichtung 3 geförderte Atemluft befeuchten kann. Angesteuert wird die Atemluftbefeuchtungseinrichtung 36 durch die Atemluftsteuereinrichtung 34', die vorzugsweise ein Messsignal von einem Feuchtesensor 37 erhält, der in dem befeuchteten Atemluftstrom 4 angeordnet ist und mit der Atemluftsteuereinrichtung 34' verbunden ist. Optional ist die Atemluftsteuereinrichtung 34', ebenso wie die im Zusammenhang mit Figur 2 beschriebene Atemluftsteuereinrichtung 34, mit der Atemluftfördereinrichtung 31 verbunden, um den Ventilator zu steuern. Ferner steht vorzugsweise die Atemluftsteuereinrichtung 34' in Verbindung mit der Steuereinrichtung 14 der Aerosolerzeugungseinrichtung 1, um die Befeuchtungseinrichtung 36 in den Phasen anzusteuern, in denen keine Aerosolerzeugung, d.h. keine Abgabe von Flüssigkeitströpfchen 2 durch die Aerosolerzeugungseinrichtung 1 stattfindet. In diesen Phasen ist das Anfeuchten der Atemluft besonders vorteilhaft, während eine zu feuchte Atemluft während der Medikamentengabe, d.h. bei angesteuertem Aerosolgenerator 11 verhindert, dass die Tröpfchen 2 durch die zugeführte Atemluft 4 weiter abgetrocknet werden.

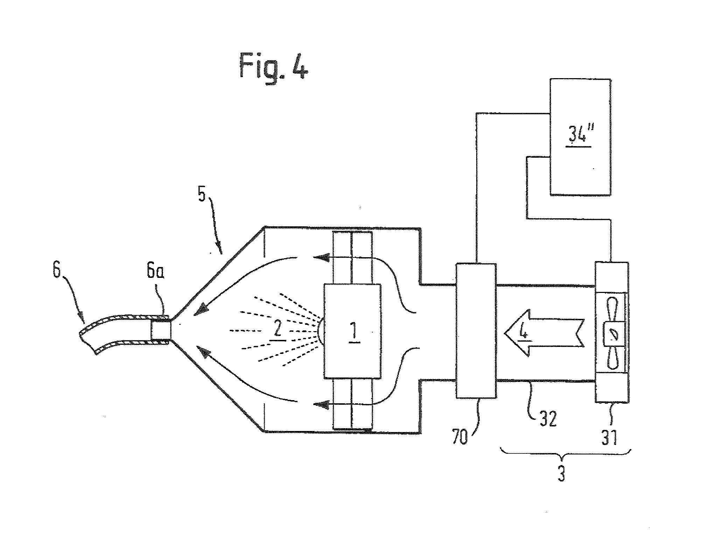

Figur 4 zeigt ein viertes Ausführungsbeispiel einer erfindungsgemäßen Inhalationstherapievorrichtung, jedoch in einer vereinfachten Darstellung, um die Komplexität zu reduzieren. Dennoch umfasst auch das vierte Ausführungsbeispiel vorzugsweise die Komponenten, die zuvor insbesondere im Zusammenhang mit dem ersten Ausführungsbeispiel ausführlich geschildert wurden. Insofern wird auf die Beschreibung des ersten bis dritten Ausführungsbeispiels Bezug genommen, ohne dass aber sämtliche Komponenten in Figur 4 erneut gezeigt und mit Bezugszeichen versehen sind.

Im Vordergrund des vierten Ausführungsbeispiels steht die in Figur 4 gezeigte Atemluftpulsationseinrichtung 70, die auf die Atemluftströmung 4 einwirkt, um der Atemzuluft zur Verneblerkammer 5 Druckschwingungen zu überlagern. Auf diese Weise wird die Atemluft, die zusammen mit den Flüssigkeitströpfchen aus der Verneblerkammer 5 über die Intubierungseinrichtung 6 dem Frühgeborenen/Kleinkind zugeführt wird, mit einer überlagerten Vibration (Druckschwankungen/-schwingungen) zugeführt, was zur Rekrutierung zusätzlicher Lungenbereiche führen kann. Rekrutierung bedeutet in diesem Zusammenhang, dass eine Lungenregion, welche vorher nicht am Gasaustausch teilgenommen hat, aktiviert wird.

Die Pulsationseinrichtung 70, die in Figur 4 schematisch gezeigt ist, kann auf verschiedene Weise realisiert werden, beispielsweise durch ein steuerbares oder selbsttätiges Ventilsystem oder die Aufbringung eines Wechseldrucks, der von einem Kolbenkompressor hervorgerufen wird. Gesteuert wird die Pulsationseinrichtung 70 durch eine Atemluftsteuereinrichtung 34", die vorzugsweise auch mit der Fördereinrichtung 31 der Atemluftstromerzeugungseinrichtung 3 verbunden ist und diese steuert. Offensichtlich kann die Atemluftsteuereinrichtung 34" gemäß dem vierten Ausführungsbeispiel auch mit der Steuereinrichtung 14 der Aerosolerzeugungseinrichtung 1 verbunden sein, um eine Steuerung der Pulsationseinrichtung 70 in Abhängigkeit von bzw. unter Berücksichtigung der Erzeugung des Aerosols 2 durchzuführen.

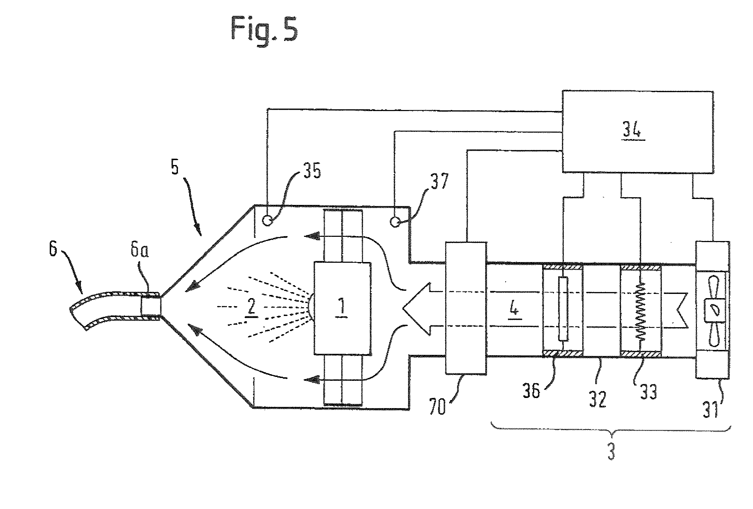

Figur 5 zeigt ein fünftes Ausführungsbeispiel der erfindungsgemäßen Inhalationstherapievorrichtung, bei dem die aus den Figuren 2 bis 4 ersichtlichen zusätzlichen Komponenten in einer Vorrichtung zusammengefasst sind. Dadurch soll beispielhaft zum Ausdruck gebracht werden, dass die zusätzlich zu den Grundkomponenten vorhandenen Zusatzeinrichtungen auch in unterschiedlicher Kombination gemeinsam eingesetzt werden können.

Diesem Gedanken folgend zeigt Figur 5 ein Ausführungsbeispiel, bei dem neben den Grundkomponenten, die im Zusammenhang mit Figur 1 ausführlich beschrieben wurden und die aus diesem Grunde hier nicht erneut vollständig erläutert und in Figur 5 gezeigt werden, gemäß Figur 5 die Atemluftheizeinrichtung 33, die Atemluftbefeuchtungseinrichtung 36 und die Pulsationseinrichtung 70 gemeinsam vorgesehen sind. Die Steuerung der Atemluftheizeinrichtung 33, der Atemluftbefeuchtungseinrichtung 36 und der Pulsationseinrichtung 70 übernimmt die Atemluftsteuereinrichtung 34, die dazu mit den Einrichtungen 33, 36 und 70 verbunden ist. Ferner ist vorzugsweise die Atemluftsteuereinrichtung 34 mit der Fördereinrichtung 31 der Atemluftstromerzeugungseinrichtung 3 verbunden. Messsignale erhält die Atemluftsteuereinrichtung 34 von dem(n) Temperatursensor(en) 35, 35', 35" und dem Feuchtesensor 37, die beide in Figur 5 schematisch dargestellt sind. Nicht in Figur 5 dargestellt ist eine Verbindung zwischen der Atemluftsteuereinrichtung 34 und der in der Aerosolerzeugungseinrichtung 1 vorhandenen Steuereinrichtung 14 (vgl. Figuren 1 bis 3), die zweckdienlicherweise vorgesehen ist, um die Ansteuerung der Einrichtungen 33, 36 und 70 sowie 31 unter Berücksichtigung der Aerosolerzeugung durch die Aerosolerzeugungseinrichtung 1 durchzuführen.

Wie sich aus allen Figuren und den darin gezeigten Ausführungsbeispielen ergibt, umfasst die erfindungsgemäße Inhalationstherapievorrichtung eine Aerosolerzeugungseinrichtung 1, eine Atemluftstromerzeugungseinrichtung 3 und einer Verneblungskammer 5, an die eine schlauchförmige Intubiereinrichtung 6 angeschlossen ist. Die Verneblungskammer 5 weist nicht nur einen sich verjüngenden und in die Intubiereinrichtung mündenden Bereich auf, sondern erlaubt auch die Durchmischung der zugeführten Atemluft 4 und der Flüssigkeitströpfchen 2, die von der Atemluftstromerzeugungseinrichtung 3 bzw. der Aerosolerzeugungseinrichtung 1 der Verneblungskammer 5 zugeführt werden. In einer besonders vorteilhaften Gestaltung, die in den Figuren 1 bis 5 gezeigt ist, wonach die Aerosolerzeugungseinrichtung 1 in der Verneblerkammer 5 angeordnet ist, stellt sich um die Aerosolerzeugungseinrichtung 1 aufgrund der Anordnung der Atemluftstromerzeugungseinrichtung 3 eine um die Aerosolerzeugungseinrichtung 1 herum verlaufende Atemluftströmung 4 ein. Dies führt vorteilhafterweise dazu, dass die Flüssigkeitströpfchen 2 von der Atemluftströmung mantelartig umschlossen werden und so eine Deposition in der Verneblungskammer 5 quasi ausgeschlossen ist, ohne dass eine gute Durchmischung der Flüssigkeitströpfchen der Atemluft 4 beeinträchtigt wird. Durch die sich verjüngende Zuführung der Atemluftströmung mit den darin geförderten Flüssigkeitströpfchen erfolgt eine optimale Zuführung des Flüssigkeitströpfchen/Atemluft-Gemisches in die Intubiereinrichtung. Trotz der möglicherweise gering erscheinenden Abmessungen, die für die Intubiereinrichtung erforderlich sind, tritt eine Deposition der Flüssigkeitströpfchen praktisch nicht auf, so dass das vernebelte Fluid, insbesondere ein vernebeltes Surfactant nahezu vollständig in die Lunge eines Frühgeborenen/Kleinkindes appliziert werden kann. Dazu trägt in erheblichem Maße auch die Gestaltung des Intubierschlauchs bei, dessen für die Intubation vorgesehenes Ende so gestaltet ist, dass eine Abgabe erst hinter den filternden Atemwegsbereichen (siehe oben) erfolgt.

In der vorangegangenen Beschreibung der Erfindung wurde insbesondere auf die Verabreichung eines Surfactants Bezug genommen. Jedoch ist der Beschreibung der Erfindung ebenfalls zu entnehmen, dass sich ein Inhalationstherapiesystem gemäß der Erfindung grundsätzlich für die inhalative Applikation von Medikamenten jedweder Art eignet, um Neugeborenen und Frühchen eine topische oder systemische Arzneitherapie mit dem erfindungsgemäßen Inhalationstherapiesystem zukommen zu lassen, dadurch gekennzeichnet, dass Körperfunktionen und/oder ein unnatürlicher oder krankhafter Zustand wieder in einen Normalzustand überführt und Leiden gelindert oder geheilt werden.

Die Arzneitherapie ist dadurch gekennzeichnet, dass mit dem erfindungsgemäßen Inhalationstherapiesystem besonders vorteilhaft Medikamente jedweder Art und Klasse aus tierischem, bakteriellem, menschlichem oder synthetischem Material inhalativ appliziert werden können, wie z.B.
- Lungensurfactant (wie z.B. Surfaxin),
- antientzündliche Mittel, wie Steroide (Ciclesonid, Fluticason),
- nicht-steroidale Entzündungshemmer (wie z.B. Ibuprofen, Celecoxib),
- Betamimetika (wie z.B. Indacaterol, Formoterol, Levalbuterol),
- Anticholinergika (wie z.B. Thiotropium, Glycopyrrolate, Ipratropium),
- Phosphodiesterasehemmer (wie z.B. Sildenafil, Vardenafil, Tadalafil),
- Endothelinantagonisten (wie z.B. Bosentan, Sixtasentan, Tezosentan),
- Leukotrienantagonisten (wie z.B. Montelukast),
- Diuretika (wie z.B. Furosemid, Amilorid),
- Immunmodulatoren (wie z.B. Cyclosporin, Mofetil, Sirolimus, Tacrolimus),
- Blutdrucksenkende Mittel (wie z.B. Statine, Sartane, Calcium- und Angiotension Antagonisten),
- Mucolytika (wie z.B. Dornase alpha, Bromhexin, Ambroxol, Acetycystein),
- Antibiotika verschiedenster Klassen, wie Chinolone, Makrolide, Cephalosporine, Aminoglycoside, Ketolide, Peptide und Proteine,
- Interferone,
- Immunglobuline,
- Prostine,
- Antimykotika,
- Antivirale Mittel,
- Heparin und Heparinoide,
- Cytostatika,
- körpereigene Substanzen, die durch einen Gendefekt wie z.B. Mucoviszidose oder Krankheitszustand nicht in ausreichender Menge im Körper verfügbar sind, wie z.B. Alpha-Antitrypsin, Interferone, Insulin, etc.

Diese Substanzen können in Form von Säuren oder Basen als pharmazeutisch gebräuchliche Salze oder Komplexe, Prodrugs oder ihrer optisch aktiven Antipoden, Stereoisomere, Enantiomere, allein oder in Kombinationen eingesetzt werden.

Besonders geeignete Medikamentenformulierungen sind dadurch gekennzeichnet, dass sie als wässrige Zubereitungen in Volumina von 0.3 bis 10 ml und besonders bevorzugt in Volumina von 0.5 bis 5 ml vernebelbar sind und mit dem erfindungsgemäßen Inhalationstherapiesystem ein Aerosol mit einem Medianen Massendurchmesser (MMD) von kleiner 5 µm und besonders bevorzugt kleiner 3.5 µm mit einer schmalbandigen Partikelverteilung erzeugbar ist, die durch eine geometrische Standardabweichung von kleiner 2 und besonders bevorzugt kleiner 1.6 ausgezeichnet ist, wodurch eine in-vitro Lungendosis in einem Cast-Modell > 20% und besonders bevorzugt > 25% erreicht wird, die über derjenigen herkömmlicher Inhalationstherapiesysteme mit Jet-Düsen-Vernebler liegt.

Die inhalative Medikamententherapie mit dem innovativen Inhalationstherapiesystem ist dadurch gekennzeichnet, dass damit respiratorische Krankheitsbilder, wie frühkindliche Lungenerkrankungen, Lungendistressysmptome, Asthma, obstruktive Bronchitis, bakterielle und nicht bakterielle Entzündungen, Husten, pulmonaler Bluthochdruck, parenchymale Erkrankungen, genetische Defekte wie z.B. Mucoviszidose, frühkindliche Diabetes, etc. bevorzugt behandelt werden können.

Aus einem anderen Blickwinkel kann das Inhalationstherapiesystem gemäß der Erfindung als geeignet angesehen werden für die verbesserte inhalative Applikation von Medikamenten mit einem innovativen Verneblerkonzept, das insbesondere auf die Bedürfnisse der Behandlung von Frühchen und auch normalen Kleinkindern abgestimmt ist.

Für die Erreichung einer besonders hohen Effizienz kann die Applikation von Medikamenten unter Anwendung verschiedener Applikationsstrategien erfolgen. Da die sich für den Einzelfall anbietende Applikationsstrategie von verschiedenen Randbedingungen, z.B. von dem zu applizierenden Medikament bzw. der zu applizierenden Medikamentenkombination, von den konstruktiven Details des Inhalationstherapiesystems, von der zu therapierenden Patientengruppe etc. abhängt, wird im Folgenden ein repräsentatives Beispiel erläutert, das einen durch eine Applikationsstrategie beeinflussbaren Bereich genauer darstellt. Vor dem Hintergrund experimenteller Ergebnisse, die aufzeigen, dass bei einer zu hohen Outputrate des Aerosolerzeugers die Effizienz niedriger liegt als bei einer verringerten Outputrate, bietet sich eine Applikationsstrategie an, die diesen Ergebnissen Rechnung trägt. Wesentlich für die hier beispielhaft erläuterte Applikationsstrategie ist eine Verringerung bzw. Anpassung der Outputrate des Aerosolerzeugers, wodurch die Applikationsstrategie zu einer erhöhten Effizienz führt. Die Verringerung/Anpassung der Outputrate kann durch wechselndes Ein/Aus-Schalten des Aerosolerzeugers auf besonders flexible Art und Weise erreicht werden, da durch Anpassung/Änderung der Ein/Aus-Schaltphasen die Outputrate in weiten Bereichen festgelegt (eingestellt) werden kann.

Um die Wirksamkeit der Therapie nicht zu gefährden oder verzögert eintreten zu lassen, kann in einer speziellen Gestaltung der Applikationstherapie der Phase mit verringerter Outputrate eine Phase mit erhöhter Outputrate vorangestellt werden. Dabei wird in Kauf genommen, dass die Effizienz während dieser vorgeschalteten Phase geringer ist und eine größere Medikamentenmenge eingesetzt werden muss als bei einer optimierten Outputrate. Durch die bewusst erhöhte Outputrate mit geringerer Effizienz kann aber erreicht werden, dass die angestrebte Wirkung des Medikaments zu einem gewünschten früheren Zeitpunkt einsetzt als dies bei einer Applikation bei verringerter Outputrate von Anfang an erzielbar wäre. Eine reduzierte Outputrate ist optimierend hinsichtlich der Wirkstoffausnutzung (=Effizienz der Applikation, minimierte Wirkstoffverluste im System) einsetzbar. Es ist offensichtlich, dass durch geeignete Applikationsstrategien sehr flexibel eine wirksame Therapie mit einer hohen Effizienz und Wirksamkeit verbunden werden kann.

Am Beispiel des bereits erwähnten Surfactants soll dieser Aspekt nochmals beispielhaft verdeutlicht werden. Wird in einer ersten Phase der Applikationsstrategie das Surfactant dem Frühgeborenen mit einer hohen Outputrate mit Hilfe des erfindungsgemäßen Inhalationstherapiesystems verabreicht, wird rasch die stabilisierende Wirkung des Surfactants auf die Lunge und einer Verbesserung der Lungenfunktion erreicht. Hat sich die Lungenfunktion des Frühgeborenen stabilisiert, kann in einer zweiten Phase der Applikationsstrategie die Outputrate mit dem Ziel verringert werden, den Einsatz des Surfactants nun hinsichtlich der Effizienz zu optimieren, wodurch eine bessere Ausnutzung des wertvollen Wirkstoffs, bei dem es sich nicht nur um das zuvor angesprochene Surfactant handeln muss, erreicht wird.

Ganz offensichtlich eignet sich ein Membranvernebler als Aerosolerzeuger in herausragender Weise für die Umsetzung der zuvor erläuterten Applikationsstrategie. Denn ein Membranvernebler lässt sich in besonders geeigneter Weise durch das Ansteuersignal an den die Membran in Schwingungen versetzenden Piezo-Schwinger ein- bzw. ausschalten. Der gepulste Betrieb, in dem sich Aerosolerzeugungsphasen und Ruhephase abwechseln, ist problemlos und sehr präzise zu realisieren. Auf diesem Weg ist eine Optimierung für jede sinnvolle/erforderliche Applikationsstrategie realisierbar und optimierbar.

## Patentansprüche

1. Inhalationstherapievorrichtung mit
- einer Aerosolerzeugungseinrichtung (1) mit einem Aerosolgenerator (11) der eine Membran (13) und einen die Membran für die Vernebelung eines Fluids und Bereitstellung von Flüssigkeitströpfchen in Schwingung versetzenden Piezoschwinger aufweist,
- einer Atemluftstromerzeugungseinrichtung (3) für die Erzeugung einer Atemluftströmung (4) und Förderung der Atemluftströmung in eine Zuführleitung (32);
- einer Vernebelungskammer (5),
- wobei die Flüssigkeitströpfchen (2) und die Atemluftströmung (4) der Verneblungskammer (5) zuführbar sind, so dass sich die Flüssigkeitströpfchen und die Atemluft in der Verneblungskammer (5) zu einem Flüssigkeitströpfchen/Atemluft-Gemisch mischen, und
- die Verneblungskammer (5) einen sich verjüngenden Bereich (52) umfasst, der in eine Austrittsöffnung (53) mit einem Stutzen (54) zum Anschluss einer schlauchförmigen Intubierungseinrichtung (6) mündet, um das Flüssigkeitströpfchen/Atemluft-Gemisch aus der Verneblungskammer auszugeben und mittels der schlauchförmigen Intubierungseinrichtung (6) zu applizieren,
**dadurch gekennzeichnet, dass**
- die Vernebelungskammer (5) eine Atemluftzuführöffnung (51) aufweist, an der die Zuführleitung (32) angeordnet ist und die Aerosolerzeugungseinrichtung (1) in der Verneblungskammer (5) angeordnet ist, so dass sich beim Eintreten der Atemluft (4) in die Vernebelungskammer eine verwirbelte Atemluftströmung einstellt, wobei die Abgabe der Flüssigkeitströpfchen unmittelbar in die Verneblungskammer erfolgt und die Aerosolerzeugungseinrichtung (1) von der von der Atemluftstromerzeugungseinrichtung zugeführten Atemluft (4) umströmt wird, so dass die Flüssigkeitströpfchen (2) von der Atemluftströmung (4) mantelartig umschlossen werden, wobei eine Halterungseinrichtung (15) mit mehreren radial verlaufenden Stützelementen zum Fixieren der Aerosolerzeugungseinrichtung in der Verneblungskammer (5) und Durchtrittsöffnungen (16) für die der Verneblungskammer (5) zugeführte Atemluft vorgesehen ist.

2. Inhalationstherapievorrichtung nach einem der vorangegangenen Ansprüche, **dadurch gekennzeichnet, dass** die Aerosolerzeugungseinrichtung (1) ein Reservoir (12) für die Bevorratung der zu vernebelnden Flüssigkeit aufweist.

3. Inhalationstherapievorrichtung nach Anspruch 2,
**dadurch gekennzeichnet, dass** die Aerosolerzeugungseinrichtung (1) eine Fluidheizeinrichtung für die in dem Reservoir der Aerosolerzeugungseinrichtung bevorratete Flüssigkeit aufweist.

4. Inhalationstherapiesystem nach einem der vorstehenden Ansprüche, wobei der Aerosolgenerator (11) so angeordnet ist, dass sich die Flüssigkeitströpfchen auf den sich verjüngenden Bereich (52) der Verneblungskammer (5) zu bewegen.

5. Inhalationstherapiesystem nach einem der vorstehenden Ansprüche, wobei in der Vernebelungskammer (5) Mittel (15, 16) vorgesehen sind, die die verwirbelte Atemluftströmung in eine gerichtete Strömung des Flüssigkeitströpfchen/Atemluft-Gemisches überführen.

## Claims

1. Inhalation therapy apparatus having
- an aerosol generating device (1) with an aerosol generator (11) which has a diaphragm (13) and a piezo oscillator which sets the diaphragm in oscillation for nebulisation of a fluid and preparation of liquid droplets,
- a respiratory air stream generating device (3) for generating a respiratory air flow (4) and conveying the respiratory flow into a delivery pipe (32);
- a nebulisation chamber (5),
- wherein the liquid droplets (2) and the respiratory air flow (4) can be delivered to the nebulisation chamber (5), so that the liquid droplets and the respiratory air mix in the nebulisation chamber (5) to form a liquid droplet/respiratory air mixture, and
- the nebulisation chamber (5) comprises a tapering region (52) which leads into an outlet opening (53) with a connection piece (54) for connecting a hose-like intubation device (6), in order to dispense the liquid droplet/respiratory air mixture from the nebulisation chamber and apply it by means of the hose-like intubation device (6),
**characterised in that**
- the nebulisation chamber (5) has a respiratory air delivery opening (51) on which the delivery pipe (32) is mounted and the aerosol generating device (1) is mounted in the nebulisation chamber (5) so that when the respiratory air (4) enters the nebulisation chamber a turbulent respiratory air flow arises, wherein the liquid droplets are dispensed directly into the nebulisation chamber and the respiratory air (4) delivered by the respiratory air stream generating device flows round the aerosol generating device (1), so that the liquid droplets (2) are surrounded by the respiratory air flow (4) like a sheath, wherein a supporting device (15) with several radially extending support elements for fixing the aerosol generating device in the nebulisation chamber (5) and through-openings (16) for the respiratory air delivered to the nebulisation chamber (5) is provided.

2. Inhalation therapy apparatus according to any of the preceding claims, **characterised in that** the aerosol generating device (1) has a reservoir (12) for storing the liquid to be nebulised.

3. Inhalation therapy apparatus according to claim 2, **characterised in that** the aerosol generating device (1) has a fluid heating device for the liquid stored in the reservoir of the aerosol generating device.

4. Inhalation therapy system according to any of the preceding claims, wherein the aerosol generator (11) is disposed in such a way that the liquid droplets move towards the tapering region (52) of the nebulisation chamber (5).

5. Inhalation therapy system according to any of the preceding claims, wherein in the nebulisation chamber (5) are provided means (15, 16) which convert the turbulent respiratory air flow to a directional flow of the liquid droplet/respiratory air mixture.

## Revendications

1. Dispositif de thérapie par inhalation, comprenant
- un dispositif de génération d'aérosol (1) avec un générateur d'aérosol (11) pourvu d'une membrane (13) et d'un oscillateur piézo-électrique faisant vibrer la membrane pour atomiser un fluide et préparer des gouttelettes de liquide,
- un dispositif de génération de flux d'air respirable (3) pour générer un flux d'air respirable (4) et refouler le flux d'air respirable dans un conduit d'amenée (32) ;
- une chambre d'atomisation (5),
- où les gouttelettes de liquide (2) et le flux d'air respirable (4) peuvent être amenés dans la chambre d'atomisation (5), si bien que les gouttelettes de liquide et l'air respirable forment dans la chambre d'atomisation (5) un mélange gouttelettes de liquide/air respirable, et où
- la chambre d'atomisation (5) comprend une région (52) qui se rétrécit, débouche dans une ouverture de sortie (53) avec un raccord (54) pour connexion à un dispositif d'intubation (6) tubulaire, afin d'évacuer le mélange gouttelettes de liquide/air respirable de la chambre d'atomisation et l'administrer au moyen du dispositif d'intubation (6) tubulaire,
**caractérisé en ce que**
- la chambre d'atomisation (5) comporte une ouverture d'amenée d'air respirable (51) contre laquelle est monté le conduit d'amenée (32), et le dispositif de génération d'aérosol (1) est disposé dans la chambre d'atomisation (5), si bien qu'un flux d'air respirable est rendu turbulent à l'admission de l'air respirable (4) dans la chambre d'atomisation, la délivrance des gouttelettes de liquide étant directement effectuée dans la chambre d'atomisation et l'air respirable (4) amené par le dispositif de génération de flux d'air respirable contournant le dispositif de génération d'aérosol (1), si bien que les gouttelettes de liquide (2) sont entourées par le flux d'air respirable (4) à la manière d'une enveloppe, un dispositif de support (15) à plusieurs éléments d'appui à extension radiale pour la fixation du dispositif de génération d'aérosol étant prévu dans la chambre d'atomisation (5) ainsi que des ouvertures de passage (16) pour l'air respirable amené vers la chambre d'atomisation (5).

2. Dispositif de thérapie par inhalation selon l'une des revendications précédentes, **caractérisé en ce que** le dispositif de génération d'aérosol (1) comporte un réservoir (12) de stockage pour le liquide à atomiser.

3. Dispositif de thérapie par inhalation selon la revendication 2, **caractérisé en ce que** le dispositif de génération d'aérosol (1) comprend un dispositif de chauffage de fluide pour le liquide stocké dans le réservoir du dispositif de génération d'aérosol.

4. Système de thérapie par inhalation selon l'une des revendications précédentes, où le générateur d'aérosol (11) est disposé de telle manière que les gouttelettes de liquide se déplacent vers la région (52) rétrécie de la chambre d'atomisation (5).

5. Système de thérapie par inhalation selon l'une des revendications précédentes, où des moyens (15, 16) sont prévus dans la chambre d'atomisation (5), lesquels transforment le flux turbulent d'air respirable en un flux directionnel gouttelettes de liquide/air respirable.
